# EUROPEAN PATENT APPLICATION

(11) **EP 2 982 305 A1**
(43) Date of publication of application: **10.02.2016**
(21) Application number: 14779460.6
(22) Date of filing: 27.03.2014
(51) Int. Cl.: A61B 8/08

(54) **DIAGNOSTIC ULTRASOUND APPARATUS AND ELASTICITY EVALUATION METHOD**

(30) Priority: 03.04.2013 JP 2013077701
(71) Applicant: Hitachi Aloka Medical, Ltd., Tokyo 181-8622 (JP)
(72) Inventor: YOSHIKAWA, Hideki, Tokyo 100-8280 (JP); ASAMI, Rei, Tokyo 100-8280 (JP); TABARU, Marie, Tokyo 100-8280 (JP)
(74) Representative: MERH-IP Matias Erny Reichl Hoffmann Patentanwälte PartG mbB
(86) International application number: PCT/JP2014/058806
(87) International publication number: WO 2014/162966

(57) **Abstract**

Provided is an ultrasonic diagnostic apparatus provided with an elasticity evaluation technology that an error caused by a tissue structure has been reduced. The ultrasonic diagnostic apparatus that measures a velocity of a shear wave that propagates in a test object by utilizing an ultrasonic wave to evaluate elasticity of the aforementioned test object transmits and receives a first ultrasonic wave to the test object to detect a position and a size of the tissue structure of the test object and to automatically decide a measurement region except the tissue structure, transmits a second ultrasonic wave to the measurement region to make it generate the shear wave, and transmits and receives a third ultrasonic wave to the measurement region to measure an amount of displacement in association with propagation of the shear wave and to calculate the shear wave velocity by utilizing this displacement amount.

## Description

### Technical Field

The present invention relates to an ultrasonic diagnostic apparatus. In particular, it relates to a technology for evaluating elasticity of a living body, utilizing acoustic radiation force.

### Background Art

An image display device for medical use represented by ultrasonic imaging, MRI (Magnetic Resonance Imaging) and X-ray CT (Computed Tomography) is widely utilized as a device that presents information in the living body that cannot be visually observed in the form of a numerical value or an image. Above all, the image display device utilizing an ultrasonic wave is provided with a high time resolution in comparison with other devices and has such a performance that the beating heart can be imaged with no bleeding.

The ultrasonic wave that propagates in the living body is broadly divided into a longitudinal wave and a transverse wave and many technologies (a technology for visualizing a tissue form and a technology for measuring a blood flow velocity) loaded on products mainly utilize information of the longitudinal wave (about 1540 m/s in sound velocity).

In recent years, a technology for evaluating a modulus of elasticity of a tissue by utilizing the transverse wave (hereinafter, a shear wave) attracts notice and clinical use thereof is being promoted for mammary tumors and chronic liver diseases. In this technology, the shear wave is generated in the tissue to be served as a measurement object and the elasticity is evaluated from a propagation velocity thereof. A technique for generating the shear wave is broadly divided into a mechanical system and a radiation pressure system. The mechanical system is a system for generating the shear wave by applying a vibration of about 1 kHz onto the body surface by utilizing a vibrator and so forth and requires a drive device serving as a vibration source. On the other hand, in the radiation pressure system, the acoustic radiation pressure is applied into the living body by utilizing focused ultrasound that makes ultrasonic waves concentrate onto a local part in the tissue and the shear wave is generated by tissue displacement in association therewith. Either system is a technology for measuring the tissue displacement in association with propagation of the generated shear wave by utilizing the ultrasonic wave and evaluating information relevant to the hardness of the tissue.

As a prior art literature related to them, there exists, for example, Patent Literature 1 that relates to a technique for elasticity evaluation utilizing the acoustic radiation pressure.

### Citation List

### Patent Literature

PTL 1: US 8,118,744 B2

### Summary of the Invention

### Technical Problem

In the technique described in Patent Literature 1, the radiation force is generated in the tissue by utilizing the focused ultrasound so as to make the shear wave propagate in the tissue. Then, a plurality of measurement spots for performing ultrasonic wave transmission and reception are provided in a propagation direction to measure a time variation of the tissue displacement. Then, an arrival time of the shear wave at each measurement spot is measured by utilizing a result of measurement of the displacement. Then, a propagation time of the shear wave between the respective measurement spots is calculated by utilizing the arrival time at each measurement spot to measure a velocity.

However, in the technique described in the above-mentioned Patent Literature 1, in a case where there exist tissue structures such as a blood vessel, a fibrous tissue and so forth on a propagation path of the shear wave, the wave front scatters by being influenced by diffraction and refraction and the form of the wave front is disturbed. In the present technique for estimating the shear wave velocity by utilizing an amount of displacement of the tissue in association with propagation, disturbance of the wave front becomes a major cause of an increase in error in elasticity evaluation.

An object of the present invention is to provide ultrasonic diagnostic apparatus and method making highly reliable elasticity evaluation possible by reducing the influence of scattering of the wave front caused by the tissue structure.

### Solution to Problem

In order to attain the above-mentioned object, in the present invention, there is provided an ultrasonic diagnostic apparatus, the ultrasonic diagnostic apparatus including a probe that transmits and receives an ultrasonic wave and a processing unit that transmits and receives the ultrasonic wave from the probe to an inspection object and processes reception data obtained from the inspection object, wherein the processing unit detects a tissue structure of the inspection object from reception data obtained by transmitting and receiving a first ultrasonic wave from the probe to the inspection object, determines a measurement region for detecting a shear wave velocity on the basis of the tissue structure detected, transmits a second ultrasonic wave to the measurement region to make it generate a shear wave, and
calculates the shear wave velocity from reception data obtained by transmitting and receiving a third ultrasonic wave to the measurement region.

In addition, in order to attain the above-mentioned object, in the present invention, there is provided an elasticity evaluation method, the elasticity evaluation method of detecting a tissue structure of an inspection object from reception data obtained by transmitting and receiving a first ultrasonic wave from a probe that transmits and receives an ultrasonic wave to the inspection object, determining a measurement region for detecting a shear wave velocity on the basis of the detected tissue structure, transmitting a second ultrasonic wave to the measurement region to make it generate a shear wave and calculating the shear wave velocity from reception data obtained by transmitting and receiving a third ultrasonic wave to the measurement region. Advantageous Effects of Invention

According to the present invention, the highly reliable tissue elasticity evaluation can be implemented by suppressing the wave front disturbance caused by the tissue structure.

### Brief Description of the Drawings

[Figure 1]

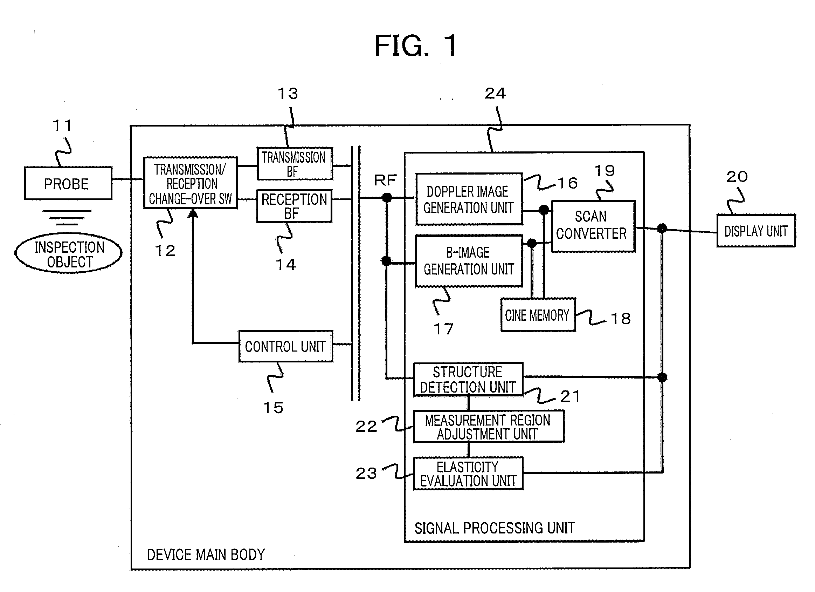

   Fig. 1 is a block diagram showing one configuration example of an ultrasonic diagnostic apparatus of a first embodiment.
[Figure 2]

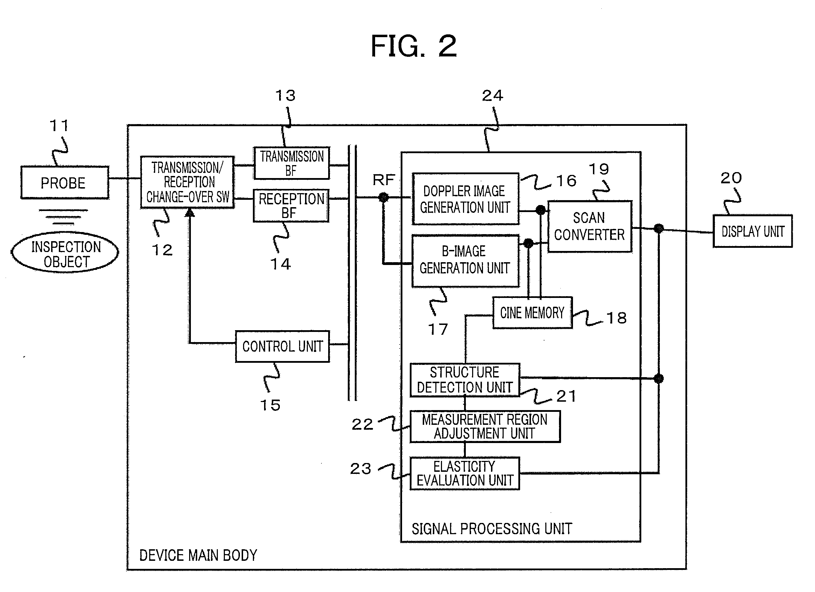

   Fig. 2 is a block diagram showing another configuration example of the ultrasonic diagnostic apparatus of the first embodiment.
[Figure 3]

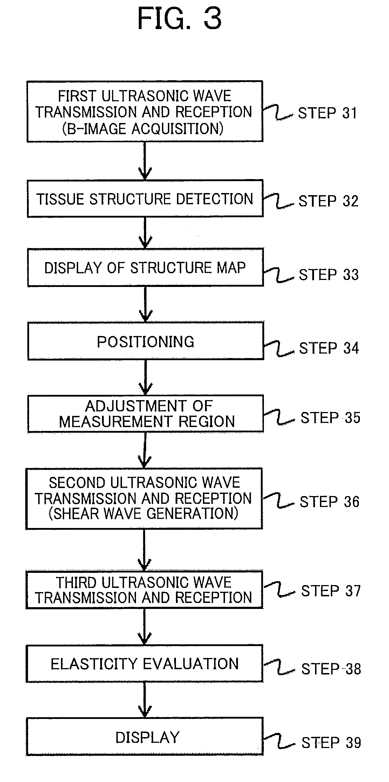

   Fig. 3 is a flowchart diagram showing the entire of processing steps of the first embodiment.
[Figure 4]

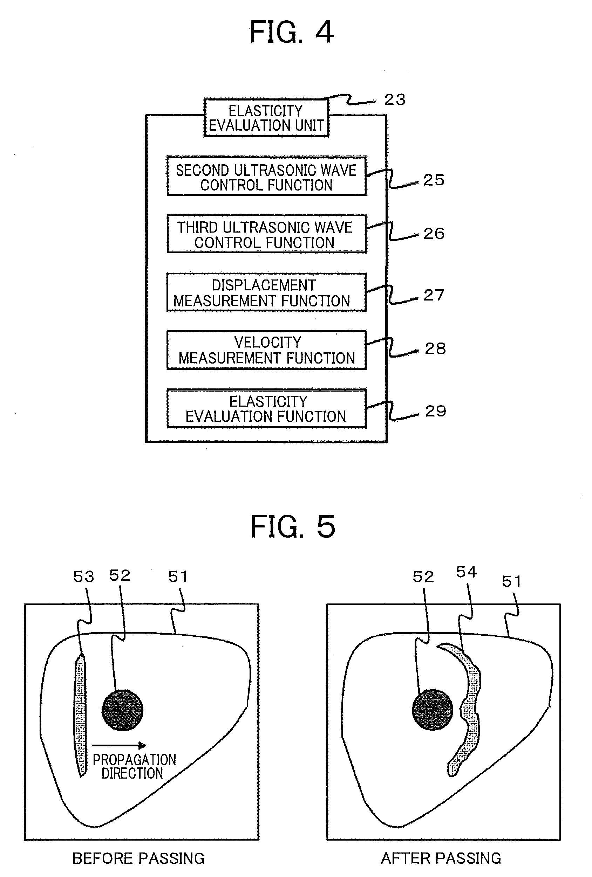

   Fig. 4 is a diagram showing a function of an elasticity evaluation unit of the first embodiment.
[Figure 5]
   Fig. 5 is diagrams for explaining a wave front disturbance caused by a tissue structure of the first embodiment.
[Figure 6]

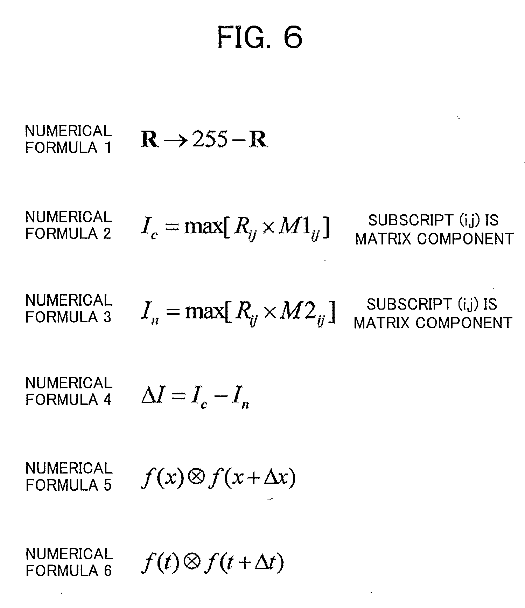

   Fig. 6 is a diagram showing numerical formulae pertaining to a processing system of the first embodiment.
[Figure 7]

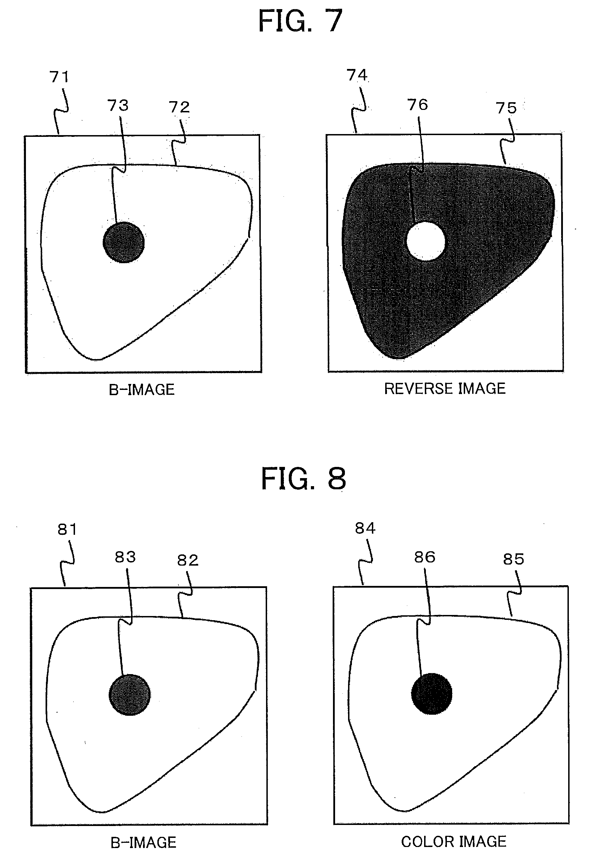

   Fig. 7 is diagrams showing one example of a structure map of the first embodiment.
[Figure 8]
   Fig. 8 is diagrams showing one more example of the structure map of the first embodiment.
[Figure 9]

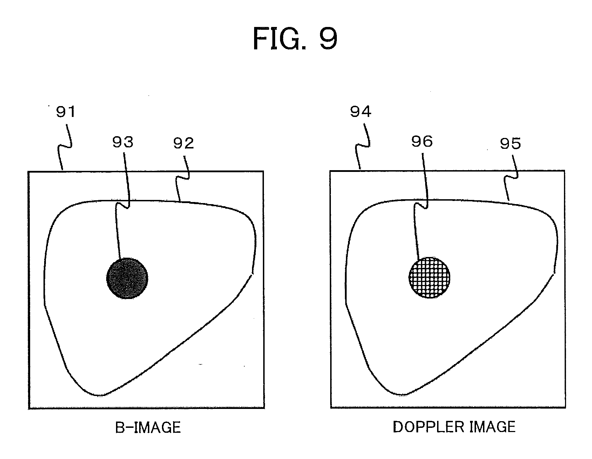

   Fig. 9 is diagrams showing one more example of the structure map of the first embodiment.
[Figure 10]

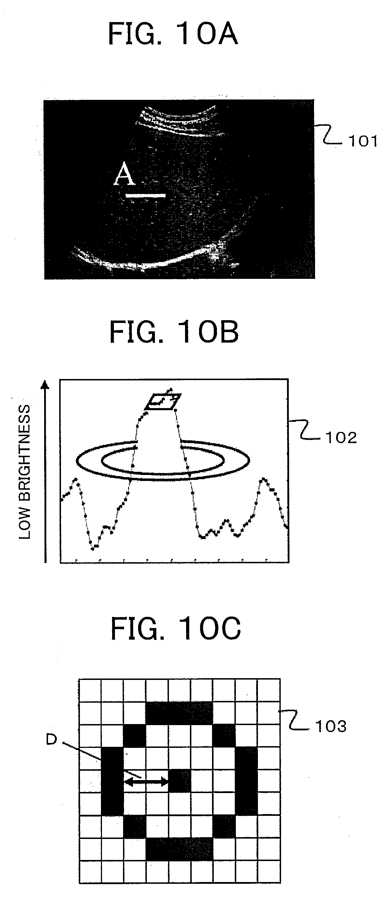

   Fig. 10 is diagrams showing one example of a structure extraction filter of the first embodiment.
[Figure 11]

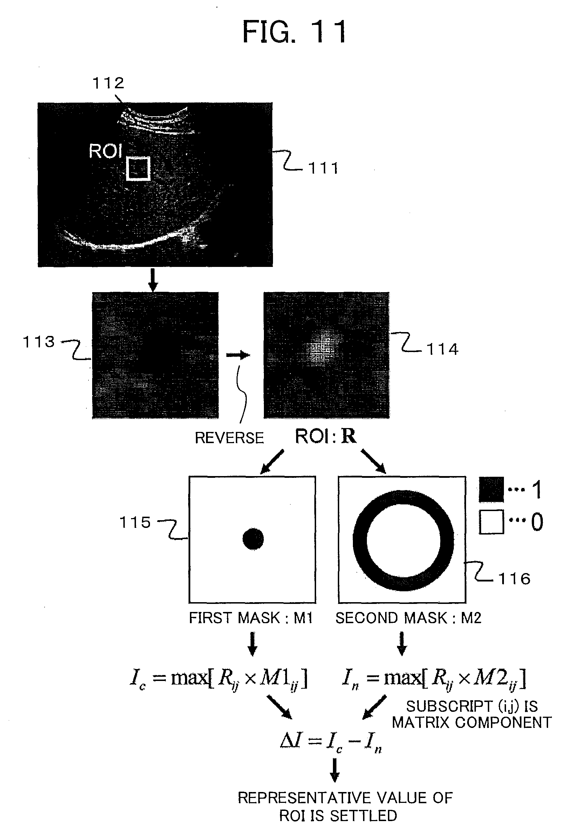

   Fig. 11 is diagrams showing one example of detection of the tissue structure of the first embodiment.
[Figure 12]

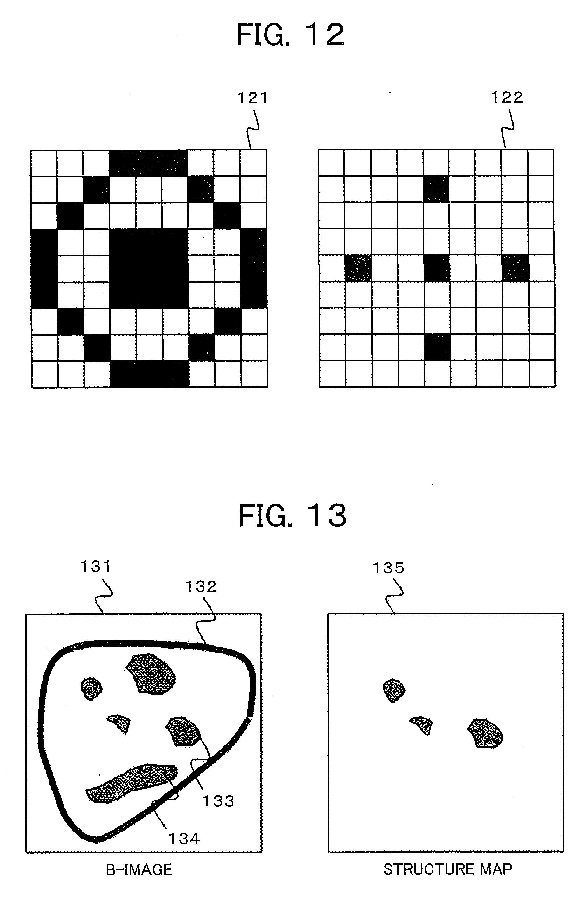

   Fig. 12 is diagrams showing one example of a mask of the first embodiment.
[Figure 13]
   Fig. 13 is diagrams showing one more example of the structure map of the first embodiment.
[Figure 14]

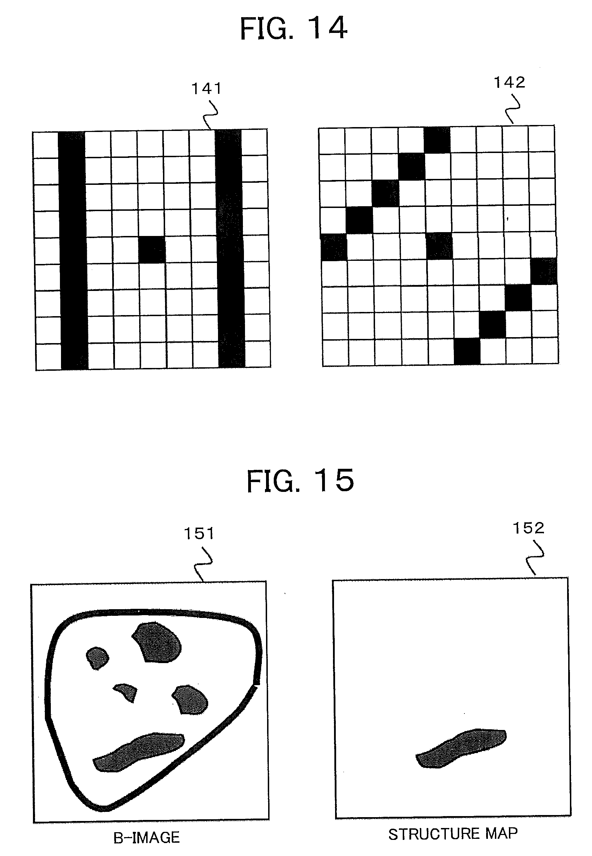

   Fig. 14 is diagrams showing one more example of the mask of the first embodiment.
[Figure 15]
   Fig. 15 is diagrams showing one more example of the structure map of the first embodiment.
[Figure 16]

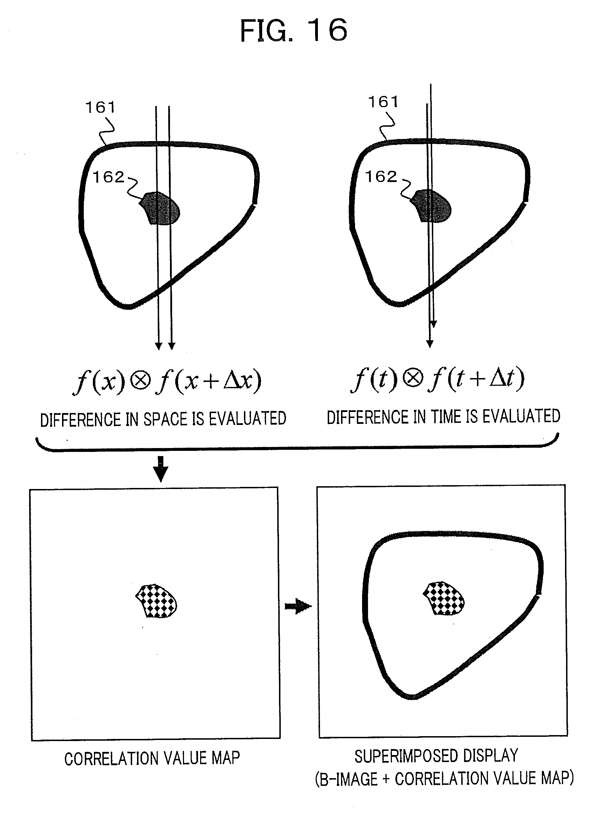

   Fig. 16 is diagrams for explaining one example of correlation arithmetic operation of the first embodiment.
[Figure 17]

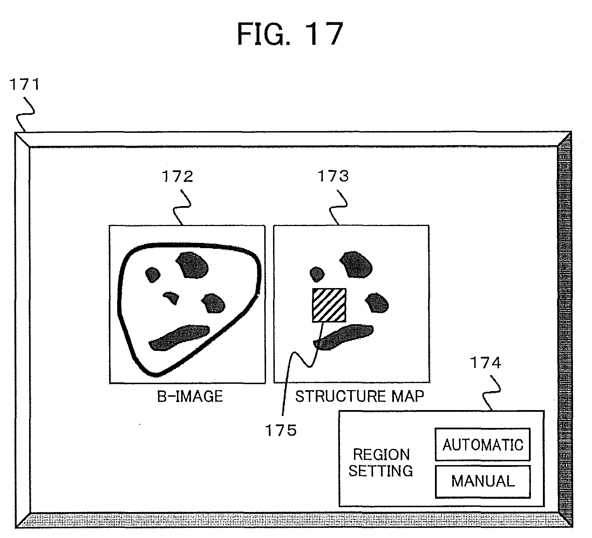

   Fig. 17 is a diagram showing one example of a GUI for setting a measurement region of the first embodiment.
[Figure 18]

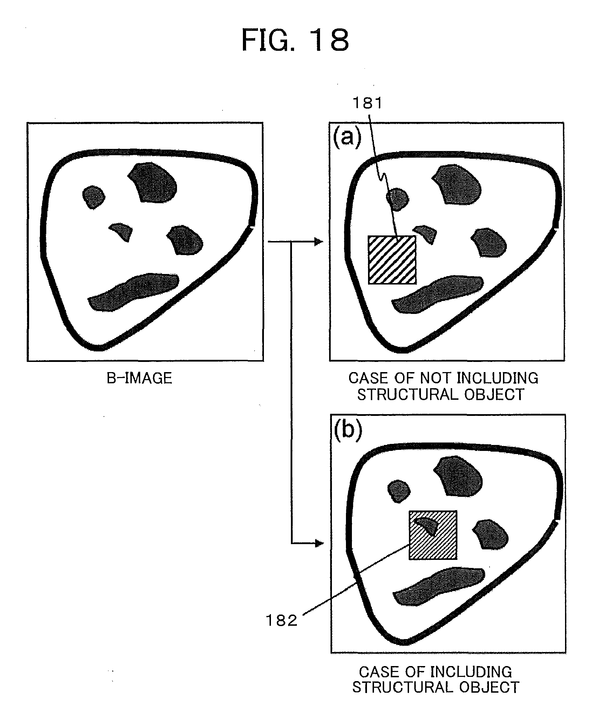

   Fig. 18 is diagrams for explaining setting of the measurement region of the first embodiment.
[Figure 19]

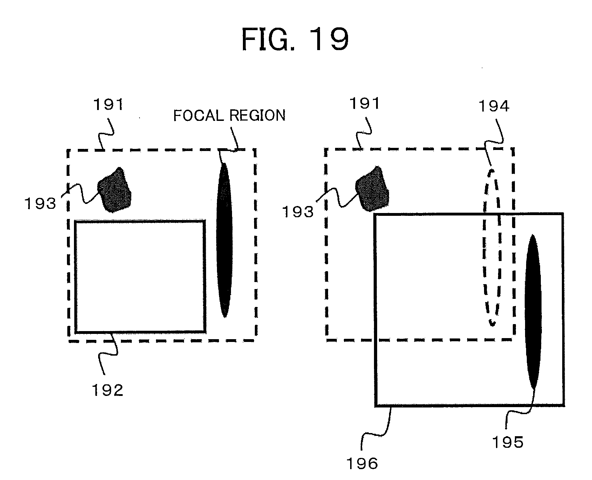

   Fig. 19 is diagrams for explaining adjustment of the measurement region of the first embodiment.
[Figure 20]

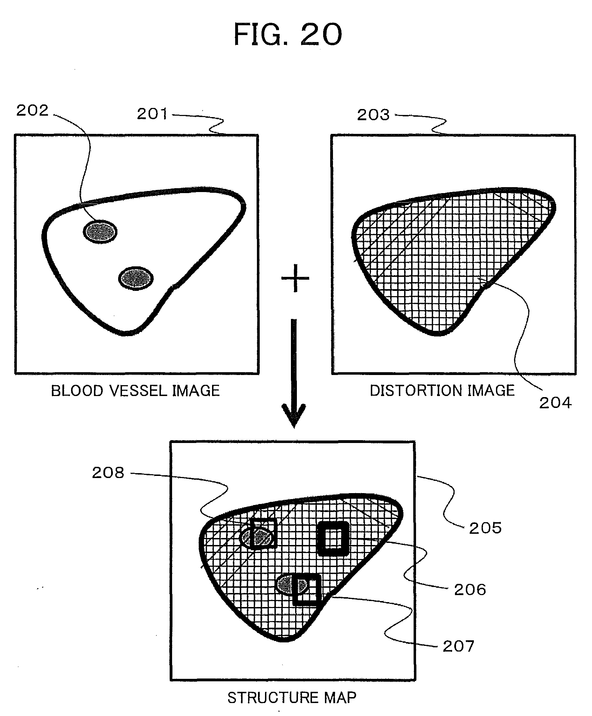

   Fig. 20 is diagrams for explaining an alert function showing the grade of appropriateness of the measurement region of the first embodiment.

### Description of Embodiments

In the following, embodiments of the present invention will be described in accordance with the drawings. Incidentally, in the present specification, it is supposed that elasticity information of the tissue refers physical property values in general of the tissue such as a strain, a shear wave velocity, a longitudinal wave velocity, a Young's modulus, a modulus of rigidity, a volume elastic modulus, a Poisson's ratio, a coefficient of viscosity and so forth. In addition, structural objects such as a blood vessel, a local fibrous tissue, a cyst and so forth that are inspection objects for measuring the shear wave velocity by the ultrasonic diagnostic apparatus according to the present invention will be referred to as the tissue structures or the structural objects

### [First Embodiment]

The first embodiment is an embodiment of an ultrasonic diagnostic apparatus provided with a probe that transmits and receives an ultrasonic wave and a processing unit that transmits and receives the ultrasonic wave from the probe to an inspection object and processes reception data obtained from the inspection object, wherein the processing unit detects a tissue structure of the inspection object from reception data obtained by transmitting and receiving a first ultrasonic wave from the probe to the inspection object, determines a measurement region for detecting a shear wave velocity on the basis of the detected tissue structure, transmits a second ultrasonic wave to the measurement region to make it generate a shear wave and calculates the shear wave velocity from reception data obtained by transmitting and receiving a third ultrasonic wave to the measurement region.

In addition, the first embodiment is an embodiment of an elasticity evaluation method, the elasticity evaluation method of detecting a tissue structure of an inspection object from reception data obtained by transmitting and receiving a first ultrasonic wave from a probe that transmits and receives an ultrasonic wave to the inspection object, determining a measurement region for detecting a shear wave velocity on the basis of the detected tissue structure, transmitting a second ultrasonic wave to the measurement region to make it generate a shear wave and calculating the shear wave velocity from reception data obtained by transmitting and receiving a third ultrasonic wave to the measurement region. Here, on the basis of the detected tissue structure is, for example, in a region except the detected tissue structure and so forth.

A shear wave velocity measuring method and a configuration example of the ultrasonic diagnostic apparatus that are the first embodiment will be described using a block diagram in Fig. 1. In the configuration of the ultrasonic diagnostic apparatus of the present embodiment, the above-mentioned processing unit is a general term of a control unit 15 and a signal processing unit 24 as described in detail later.

First, a configuration relevant to generation of RF data and image data to be utilized in the present embodiment will be described. An electric signal for transmission pulse is sent from a transmission beam former (a transmission BF) 13 for generating an ultrasonic signal for a probe 11 that has been set on the body surface of the inspection object described in Fig. 1 and transmits and receives the ultrasonic wave to the probe 11 via a digital/analog (D/A) converter illustration of which has been omitted. The electric signal input into the probe 11 is converted from the electric signal to an acoustic signal by a ceramic element installed therein and is transmitted into a test object. Transmission is conducted by a plurality of the ceramic elements and a predetermined time delay is afforded to each element such that they are focused at a predetermined depth in the test object.

The acoustic signal that has been reflected in the course of propagation in the inspection object is received again by the probe 11, is converted into the electric signal reversely to that upon transmission and is sent to a reception beam former (a reception BF) 14 that generates complex RF data from the received ultrasonic signal via an analog/digital (A/D) converter illustration of which has been omitted as reception data. Change-over between transmission and reception is conducted by a transmission/reception change-over SW 12 on the basis of control of the control unit 15 that is the processing unit. In the reception BF 14, after addition processing (phasing addition) that the time delay that has been afforded upon transmission has been taken into account has been conducted on signals received by the plurality of elements and processing such as attenuation correction and so forth has been conducted thereon, they are sent to a Doppler image generation unit 16 that generates a Doppler image indicative of a velocity and a direction of a blood flow or a B-image generation unit 17 that generates a brightness-mode(B) image indicative of form information of the tissue from the RF data in the signal processing unit 24 that is the processing unit as the complex RF data.

The RF data that is the reception data that the signal processing unit 24 has received becomes element data of a specific one line along an ultrasonic wave transmission and reception direction in image data to be finally displayed on a display unit 20. The RF data is acquired as all pieces of reception data that become constitutional elements of the image data by performing transmission and reception of the ultrasonic wave relative to the inspection object by sequentially switching them in an array direction of the ceramic elements that configure the probe 11.

Image generation processing that is generally used in prevalent ultrasonic diagnostic apparatus such as gain control, logarithmic compression, envelope detection and so forth is performed on the RF data that is the acquired reception data in the B-image generation unit 17 of the signal processing unit 24 and the B-image indicative of the form information in the inspection object is generated.

On the other hand, in the Doppler image generation unit 16 of the signal processing unit 24, blood flow information (the velocity and the direction) is calculated by correlation arithmetic operation and the Doppler image is generated. Incidentally, although an ultrasonic wave transmission and reception sequence is different from that of the system of the B-image in regard to generation of the Doppler image, the technical contents thereof are generally known contents and therefore detailed description thereof is omitted. The B-image and the Doppler image are stored into a cone memory 18. Coordinate transformation and pixel interpolation according to the kind of the probe are conducted on the generated B-image and Doppler image by a scan converter 19 and they are displayed on the display unit 20 that displays these images, evaluated images and numerical values.

As shown in Fig. 1, the signal processing unit 24 is further provided with a structure detection unit 21 that detects the tissue structure, a measurement region adjustment unit 22 that adjusts the measurement region, and an elasticity evaluation unit 23 that evaluates the elasticity of the measurement region using the shear wave velocity measured in the measurement region.

In the structure detection unit 21 of the signal processing unit 24, the tissue structure that influences scattering of the shear wave is detected by utilizing the reception data from the reception BF 14 and a structure map that the tissue structure has been emphasized is generated. That is, the structure detection unit 21 of the signal processing unit 24 creates the structure map indicative of the position and the size of the tissue structure. The created structure map is displayed on the display unit 20 to be served as support information when a practitioner designates a region for measuring the shear wave.

Incidentally, although in the configuration of the ultrasonic diagnostic apparatus shown in Fig. 1, the structure detection unit 21 generates the structure map by utilizing the FR data, it is also possible to use image data saved in the cine memory 18 as shown in a modified configuration example of the ultrasonic diagnostic apparatus in Fig. 2 as sequentially described later.

The measurement region adjustment unit 22 of the signal processing unit 24 automatically decides a region that is reduced in influence of the tissue structure by utilizing the information of the generated structure map. In a case where the measurement region includes the tissue structure, it discriminates the region that is reduced in influence of the tissue structure from the measurement region and limits a range for measuring tissue displacement.

The elasticity evaluation unit 23 of the signal processing unit 24 conducts burst wave transmission and track pulse transmission and reception in the measurement region decided by the measurement region adjustment unit 22 and evaluates information relevant to the tissue elasticity.

In the configurations of the ultrasonic diagnostic apparatus of the present embodiment shown in Figs. 1 and 2, the control unit 15 and the signal processing unit 24 that control a data flow in the device main body and processing in general can be implemented by a general computer configuration provided with a central processing unit (CPU) and a memory. That is, they can be implemented by program processing of the CPU except the cine memory 18 and the scan converter 19 that is implemented by hardware as required. Thus, in the present specification, the signal processing unit 24 and the control unit 15 will be referred to as the processing unit in combination.

Next, details of the elasticity evaluation unit 23 of the signal processing unit 24 in the configurations of the present embodiment will be described by utilizing a function explanatory diagram in Fig. 4. As described above, the present function processing can be implemented by program processing in the CPU.

As shown in the same drawing, the elasticity evaluation unit 23 is provided with a second ultrasonic wave control function 25 that settles acoustic parameters and so forth required for wave transmission such as a focusing position, a transmission angle, a burst length, a voltage, a frequency, a number of driver elements and so forth that are wave transmission conditions of a ultrasonic burst wave for generating a radiation pressure in the measurement region that has been settled in the aforementioned measurement region control, a third ultrasonic wave control function 26 that settles acoustic parameters and so forth required for wave transmission such as the focusing position, the transmission angle, a wave number, the voltage, the frequency, a transmission and reception frequency, the number of driver elements and so forth that are the wave transmission conditions of an ultrasonic pulse wave (a track pulse) for measuring the displacement of the tissue on the basis of coordinate information, a displacement measurement function 27 that measures the displacement of the tissue by utilizing the RF data output from an ultrasonic wave transmission and reception unit, a velocity measurement function 28 that measures the shear wave velocity by utilizing a result of the displacement measurement function, and an elasticity evaluation function 29 that evaluates elasticity information (here, it refers the physical property values in general relevant to deformation and flow of a substance such as the strain, the shear wave velocity, the compressional wave velocity, the Young's modulus, the modulus of rigidity, the volume elastic modulus, the Poisson's ratio, the coefficient of viscosity and so forth) of the tissue by utilizing a result of the velocity measurement function. Incidentally, an arrival time of the shear wave can be calculated from a time variation of the displacement that has been measured by the displacement measurement function 27 by utilizing a maximum value, a minimum value, an intermediate value between the maximum value and the minimum value and so forth.

In the same drawing, first, the wave transmission conditions of a push pulse are settled on the basis of position coordinates of the designated measurement region by the second ultrasonic wave control function 25. As the wave transmission conditions for effectively generating the shear wave with no influence on the living body, a focusing condition of a substantially F-number = 1 - 2 (a value that the width of the aperture has been divided by a focal depth) is appropriate and as an intensity and the burst length, ranges of 0.1 - 1 kW/cm² in intensity and 100 - 1000 µs in burst length are suited.

Here, the width of the aperture is a range of the ceramic elements to be driven in reality and takes a discrete value of an interelement spacing. Then, in order to form an ideal focal region, aperture weight (apodization) is added to the voltage to be applied to each element. Disturbance of the focal region due to the influence of diffraction is suppressed by gradually reducing the weight from the center of the aperture toward a corner. However, since the aperture weight has a drawback of reducing the intensity, in a case where an evaluation position is located on a deep part and the influence of attenuation is large, the intensity is taken in preference to region formation and the aperture weight is reduced in some cases. In addition, it is effective to set the transmission frequency in the vicinity of a center frequency of a sensitivity bandwidth of the probe 11. The transmission conditions of the push pulse are immediately sent to the transmission BF 13 via the control unit 15 and it is radiated from the probe 11 into the living body.

Then, the wave transmission conditions of the track pulse are settled by the third ultrasonic wave control function 26. The acoustic parameters such as the frequency, the wave number, the F- number and so forth become almost the same as those of the conditions when generating the image data. In a case where the inspection object is the abdominal region, the conditions of 1 - 5 MHz in frequency, 1 - 3 waves in wave number and 1 - 2 in F-number are utilized.

A reflected signal from the living body acquired by transmission of the track pulse is sent to the reception BF 14 via the probe 11 and the complex RF data is generated. The RF data is input into the displacement measurement function 27 and the tissue displacement in association with propagation of the shear wave is measured. The displacement measurement function 27 is performed by complex correlation arithmetic operation between pieces of the RF data acquired at time intervals of a pulse repetition time (PRT). In this case, in the present embodiment, a particle velocity is calculated as the displacement in unit time. Although there also exists a system of calculating an absolute value of the displacement using the RF data before wave transmission of the push pulse as a standard, the particle velocity has an effect of removing a low frequency component in association with swing of the probe and natural movement of a biological tissue and measuring the shear wave highly sensitively.

The arithmetic operation in the displacement measurement function 27 is conducted on all of the acquired RF signals and the shear wave velocity is measured by the velocity measurement function 28 on the basis of calculated displacement information.

Finally, the elasticity information (here, it refers the physical property values in general of the tissue such as the strain, the shear wave velocity, the compressional wave velocity, the Young's modulus, the modulus of rigidity, the volume elastic modulus, the Poisson's ratio, the coefficient of viscosity and so forth) of the tissue is evaluated on the basis of the measured velocity of the shear wave by the elasticity evaluation function 29.

Next, detailed contents ranging from generation of the structure map to automatic control of the measurement region by the structure detection unit 21 and the measurement region adjustment unit 22 of the signal processing unit 24 of the device of the present embodiment will be described on the basis of a flowchart in Fig. 3.

In the same drawing, in step 31, the B-image is acquired by transmission and reception of a first ultrasonic wave (the pulse signal) and the practitioner searches for an imaging plane suited for observation of a whole image of the inspection object and elasticity evaluation and measurement, for example, a plane that the tissue structure that obviously influences measurement such as a great blood vessel of about 1 cm and so forth is not included.

In step 32, the tissue structure that will cause disturbance of the wave front form of the shear wave is detected.

Fig. 5 schematically shows the wave fronts before and after passing through the tissue structure. As shown on the left side of the same drawing, a tissue structure 52 is present in an inspection object 51 and a shear wave 53 is making progress toward it. As shown on the right side of the same drawing, the wave front form of a shear wave 54 after it has passed through the tissue structure 52 is changed due to action of diffraction and refraction.

In calculation of the velocity of the shear wave, a time taken until the wave front arrives at a spatially set measurement spot is utilized. That is, in a case where the wave front form is disturbed, the arrival time is varied depending on the location of the wave front and it strongly influences the error in velocity measurement. Although as the tissue structure to be an object of detection in tissue structure detection step 32, the blood vessel through which the wave does not pass is set as a main object in characteristics of the shear wave, the local fibrous tissues and cysts are also included in the object in addition thereto. Here, for simplicity, description will be continuously made by giving the blood vessel as an example of the tissue structure.

In the following, first to fourth methods for detection of the tissue structure and structure maps obtained as a result thereof will be described. The first method is a method of utilizing human visual effects and utilizes a reverse image that the brightness (black-and-white gradation in gray scale) of the B-image generated by the B-image generation unit 17 has been reversed on the basis of the reception data obtained by transmitting and receiving the first ultrasonic wave to the inspection object as the structure map. In a case where the image data is 8-bit one, the reverse image is configured by processing of a numerical formula 1 in Fig. 6. In the numerical formula 1, R denotes the image data. Although it is general that the blood vessel is black-colored on the B-image, it is white-colored on the reverse image. Since white is a progressive color or a expansive color in comparison with black and therefore a fine blood vessel is seen emphatically or enlargedly on the reverse image, visual observation of the blood vessel is facilitated.

Examples of a B-image and a reverse image used in the first method are shown in Fig. 7. A white inspection object 72 and a black blood vessel 73 are shown on a B-image 71 shown on the left side of the same drawing. On the other hand, on a reverse image 74 shown on the right side of the same drawing, coloring is reversed and an inspection image 75 and a blood vessel 76 are respectively black-colored and white-colored. Although determination is difficult on the drawing, on the reverse image 74, the blood vessel is seen emphatically in comparison with it on the B-image 71. This effect is further emphasized in a darkroom where the ultrasonic diagnostic apparatus is generally used.

In addition, in this first method, it is also possible to use a color image that the tissue structure has been colored as the structure map in place of the reverse image 74. Although Fig. 8 shows the one that a B-image 81 on the left side of the same drawing has been changed to a color image 84 on the right side of the same drawing and a white inspection object 82 and a black blood vessel 83 are shown on the B-image 81, the visibility of the blood vessel can be improved, for example, by coloring a blood vessel 86 in red that is an expansive color and coloring an inspection object 85 in blue that is a contractive color.

Since the first method for tissue structure detection that has been described hereinabove utilizes the signal before or after scan conversion that is the B-image stored in the cine memory 18 and generated by the B-image generation unit 17, the previously described configuration corresponding to Fig. 2 is utilized as the apparatus configuration.

The second method for tissue structure detection utilizes the Doppler image generated by the Doppler image generation unit 16 as the structure map. Although it is the most direct approach in detecting the blood vessel, there are such problems that the sensitivity to a low-velocity blood flow is low and a time is required for acquisition of the image. The problem of the time is important, there is the possibility of already shifting to another imaging plane at the stage of setting the measurement region when the time is taken to display the structure map, and the object of setting the measurement region by avoiding the blood vessel cannot be attained. In a case where the aforementioned problems are solved owing to development of the imaging technology, the usability thereof as the structure map is increased. That is, it is the one that a B-image 91 on the left side of Fig. 9 has been changed to a Doppler image 94 on the right side of the same drawing and the visibility of a blood vessel 96 relative to an inspection object 95 can be improved in comparison with a blood vessel 93 relative to an inspection object 92.

Since this second method utilizes the Doppler image that has been output from the Doppler image generation unit 16 and stored into the cine memory 18, the apparatus configuration becomes the configuration corresponding to Fig. 2 similarly to the first method.

Beside this, many display forms showing the tissue structures are provided on the ultrasonic diagnostic apparatus and utilization of this display as the structure map is also included in the present embodiment. For example, a function (elastography) that a pressure is applied to the tissue from the outside and a strain that is a response thereto is measured to display a color map is also widely known. A display image relevant to the tissue elasticity and a harmonic image that only a nonlinear signal has been extracted are also useful information in evaluation for homogeneity as the tissue and serve as structure maps useful for automatic selection of the measurement region like this.

The third method for tissue structure detection utilizes filtering. This method is the one that the structure map is created by filtering having a mask modeling on the shape of the tissue structure.

Fig. 10 is diagrams for explaining this filtering. (a) on the top of Fig. 10 shows a B-image 101, (b) on the middle of Fig. 10 shows a brightness profile 102 of a line shown by A in (a) on the top and (c) on the bottom of Fig. 10 shows a mask 103 to be utilized in filtering.

This mask 103 has a ring-like shape that one central pixel and pixels separated therefrom with equal distances are set to 1 and pixel values of others are set to 0. As characteristics of the present filtering method, as shown in (b) on the middle of Fig. 10, a peak value of the brightness profile 102 is sensed by the central pixel of the mask 103 and the shape of the brightness profile 102 is sensed by pixels (a ring pixel) arranged around it like a ring. In a case where the brightness profile 102 is not circular and in a case where it has a half-value width that is wider than D, a difference between brightness values of the brightness profile 102 sensed by the central pixel (square area shown in (b)) and the ring pixel (ring area shown in (b)) is reduced. That is, a tissue structure that is circular in shape and has a half-value width that is narrower than D is increased in brightness difference of pixels sensed by the central pixel and the ring pixel. A structure map that only the tissue structural object that is the tissue structure having a desired shape has been sensed is configured by utilizing this brightness difference.

A concrete processing flow of the third method will be described by utilizing Fig. 11. In the same drawing, first, a region of interest (ROI) 112 is set on a B-image 111 to be utilized for elasticity evaluation to obtain an extracted image 113. The brightness is reversed in accordance with the numerical formula 1 in Fig. 6 to obtain a reverse image 114 and this is expressed as a matrix R. Then, a first mask 115 (M1) corresponding to the central pixel of the mask and a second mask 116 (M2) corresponding to the ring pixel of the mask are prepared. The sizes of the both masks are made the same as that of the reverse image 114.

Integration of mutual components of the reverse image 114 and the first mask 115, and the reverse image 114 and the second mask 116 is performed in accordance with a numerical formula 2 and a numerical formula 3 in Fig. 6 to calculate maximum values (I_{c} and Iₙ) thereof. Next, a differential value between I_{c} and Iₙ is calculated by a numerical formula (4) in Fig. 6. This differential value is set as a representative value of the set ROI 112.

The structure map of the third method is formed by performing the foregoing processing on all of the pixels of the B-image 111 and two-dimensionally plotting the representative value of the ROI to be set. The structure map has intense brightness on the circular blood vessel of the radius D and makes it possible to readily decide the blood vessel positon in comparison with a case of directly utilizing the B-image.

Although, in the above-mentioned description, the content is such that detection of the blood vessel has been specialized, it is also applicable to detection of the tumor, fibrillization and calcification in a sense that a tissue structural object that is a specific tissue structure is extracted by utilizing information on brightness and form. Incidentally, since the tumor, fibrillization and calcification exhibit high brightness on the image contrary to the blood vessel, reverse processing for reversing the brightness is not needed in the processing flow shown in Fig. 11.

A form 121 that the central pixel of the mask has been widened is shown on the left side of Fig. 12. In this case, redundancy is provided for the position of a peak value of a brightness profile and the blood vessel to be extracted on the structure map can be more emphasized. In addition, the right side one in Fig. 12 is a form 122 that the mask has been simplified and in this case, a reduction in processing time is implemented.

Fig. 13 shows one example of a result that filtering of the third method has been performed. An inspection object 132 and structural objects thereof 133, 134 are schematically shown on a B-image 131. In a case where a mask corresponding to the structural object 133 has been set, a structure having a shape and a size that are different from those of this, for example, the structural object 134 is not sensed by filtering and a structure map 135 is acquired.

Incidentally, the shape of the mask can be optionally changed, and a line-shaped structure can be detected from a B-image 151 on the left side of Fig. 15 as shown by a structure map 152 on the right side of the same drawing in accordance with the shape of, for example, a mask 141 on the left side of Fig. 14 or a mask 142 on the right side of Fig. 14. The above-mentioned third method copes with the data format of any of the RF data directly after the reception BF 14 or video data before and after the scan converter 19. Thus, the apparatus configuration of the third method corresponds to both of the configurations in Fig. 1 and Fig. 2.

The fourth method for tissue structure detection utilizes correlation arithmetic operation. This method is the one that the structure map is calculated by correlation arithmetic operation by utilizing a plurality of pieces of the aforementioned reception data to be acquired at spatially different positions or a plurality of pieces of reception data to be acquired at the spatially same position and at different times.

As shown on the upper left side of Fig. 16, an ultrasonic signal is transmitted and received at a specific position of the inspection object, and then the position is slightly shifted and the ultrasonic signal is again transmitted and received. Then, the correlation arithmetic operation of a numerical formula 5 in Fig. 6 is performed on each of the acquired ultrasonic signals. Since in a structure that is low in brightness such as the interior of the blood vessel or the like, spatial correlation of the brightness and the shape is lowered, a correlation value of a result of arithmetic operation is lowered.

As another method, the same result is also obtained by a system of conducting correlation arithmetic operation by performing transmission and reception of the ultrasonic signal two times at the same position by changing the time as shown on the upper right side of Fig. 16. That is, while the method described in the numerical formula 5 in Fig. 6 utilizes the spatial correlation, the method described in a numerical formula 6 in Fig. 6 utilizes correlation on the time axis.

In this fourth method, the structure map showing the blood vessel position is formed by displaying a correlation value map that a position that is low in correlation value has been emphasized in superimposition on the B-image. The fourth method copes with the data formats of both of the RF data and the video data similarly to the third method. Therefore, the apparatus configuration corresponds to those in Fig. 1 and Fig. 2. This fourth method is higher in sensitivity to the signal brightness for the RF data and is suited for the configuration of the structure map.

Incidentally, in step 34 of the flowchart in Fig. 3, the position of the measurement region is determined by utilizing the structure map created in step 33. Fig. 17 shows one example of display forms of a B-image and a structure map to be displayed on the display unit 20 or the like of the ultrasonic diagnostic apparatus in Fig. 1. A B-image 172 and a structure map 173 are being displayed side by side on a display screen 171. The practitioner can set the position of the measurement region by utilizing the structure map 173 that a relevant structural object has been extracted while grasping the whole image of the inspection object with the B-image 172 by displaying them side by side. In addition, a case of a form of displaying the translucent structure map 173 in superimposition on the B-image 172 is one example of the useful form because visual line shifting is reduced. Incidentally, on the display screen 171 in the same drawing, a region setting button 174 for selection as to whether the practitioner manually conducts region setting or conducts automatic setting is provided.

Incidentally, the practitioner can finely adjust the set measurement region freely by a generally known external input function such as a pointer and so forth. It has a function of making the practitioner recognize it as an alert in a sensibly recognizable form such as the color of the measurement region, blinking and a sound in a case where a structural object to be originally avoided has been included in the measurement region as a result of performance of fine adjustment. The practitioner can freely select information to be utilized by structure-mapping it, and in a case where a blood vessel extracted image such as, for example, the Doppler image and so forth and an elasticity image have been selected, as a region to be automatically recognized as a homogeneous region, a region that has no blood vessel structure and is homogeneous in strain distribution is selected. In association therewith, the alert by fine adjustment is, for example, multi-staged. A structure map 205 on which a blood vessel image 201 and a strain image 203 have been superimposed is shown in, for example, Fig. 20. In a case where the measurement region is to be set on it, an image that a region 206 is set as a thick frame indicating the highest appropriateness, a region 207 is set as a medium-thick frame indicating intermediate appropriateness and a region 208 is set as a thin frame indicating inappropriateness so as to make the practitioner recognize their grades of appropriateness for elasticity evaluation is displayed on the screen. Such an alert function as screen display and the sound is implemented by, for example, the measurement region adjustment unit 22 and it is effective particularly when the structure map is not displayed or is subjected to side-by-side display with no superimposition. In a case where the structure image has been superimposed on the B-image, a case where information overload sometimes occurs and overall recognition by the B-image becomes difficult is conceivable. In this case, it is necessary not to display the structure map or to subject it to side-by-side display and appropriateness grading in region setting is effectively executed only by the alert function.

One example of the form when setting a measurement region by the apparatus of the present embodiment is shown in Fig. 18. As shown in the same drawing, a measurement region 181 indicative of a range for burst wave transmission and transmission and reception of the track pulse is set on the B-image or the structure map. The practitioner sets the position for performing the elasticity evaluation manually or automatically in the form of avoiding the structural object that is being displayed on the structure map. In order to show that this measurement region 181 has been set in the form of avoiding the structural object, in other words, the correctness of the measurement region, it can be visually displayed with a difference in color, shades of color and transparency of color.

As described above, the region setting button 174 is provided on the display screen 171 in Fig. 17 and is devised so as to optionally set a manual or automatic operation. A measurement region 175 for measuring the shear wave is settled simultaneously with settlement of the position where elasticity evaluation is to be performed.

The correctness of the measurement region is made to be visually recognized by changing coloring, the density, the transparency and so forth of the measurement regions 181 and 182 to be displayed respectively in a case of not including the structural object in this measurement region 175 as shown in (a) of Fig. 18 and in a case of including the structural object as shown in (b) of Fig. 18, and thereby more optimum positioning becomes possible. Further, influence of the structural object on the wave front of the shear wave strongly depends on the wavelength of the shear wave that propagates. Therefore, in the case where the structural object is included in the measurement region 182, also a method of changing coloring and so forth in accordance with the size of that structural object becomes important information in conducting positioning.

Returning to the flowchart in Fig. 3, in step 35, adjustment of the measurement region is conducted. Adjustment of this measurement region is conducted by limiting the measurement region to the position avoiding the tissue structure or shifting the measurement region on the basis of the obtained structure map by the signal processing unit 24. That is, it is the processing step in a case where the measurement region includes the structural object when positioning in step 34. A first adjustment method is a method of limiting the measurement region. On the left side of Fig. 19, a measurement region 191 before adjustment shown by a broken line, a measurement region 192 after adjustment, a structural object 193 are shown. The measurement region 192 after adjustment is set by limiting to a range except a propagation path that includes the structural object 193. As a result, although a range for measuring the tissue displacement is narrowed and the number of data is reduced accordingly, measurement is performed without changing the measurement position that is settled in advance.

A second adjustment method is a method of shifting the measurement region. On the right side of Fig. 19, the measurement region 191 before adjustment shown by the broken line, the structural object 193, a focal region 194 before adjustment, and a measurement region 196 after adjustment, a focal region 195 after adjustment are shown. Here, the focal region refers a region where the acoustic radiation pressure is generated. In this second adjustment method, it is shifted to a position not including the structural object without changing the size of the measurement region. Specifically, a shifting amount can be minimized by calculating a vector of a central position of the measurement region and a central position of the structural object, that is, information on distance and direction and shifting it in a direction opposite to this vector. In the second adjustment method, in order to also shift the focal region, vector information is sent to the control unit 15 of the apparatus main body and control of a number of drive channels and a depth of focus that are transmission conditions of the probe 11 is performed.

Then, in step 36 in Fig. 3, transmission of the second ultrasonic wave (a burst wave) for generating the acoustic radiation pressure and the shear wave is conducted and in step 37, transmission and reception of a third ultrasonic wave for measuring the displacement amount of the tissue in association with propagation of the shear wave are performed. In step 38, elasticity evaluation of the inspection object is performed on the basis of velocity information of the shear wave and numerical values are indicated on the display unit 20 (step 39).

As above, the wave front disturbance caused by the tissue structure is suppressed and highly reliable tissue elasticity evaluation is implemented by the various embodiments described so far. As a result, a reduction in inspection time of the ultrasonic diagnostic apparatus, a reduction in load on practitioners and patients and an improvement in accuracy rate of diagnosis can be expected.

Incidentally, the present invention is not limited to the above-mentioned embodiments and includes various modified examples. For example, the above-mentioned embodiments have been described in detail for better understanding of the present invention and it is not necessarily limited to those provided with all of the configurations described above. In addition, a part of a configuration of one embodiment can be replaced with a configuration of another embodiment and a configuration of another embodiment can be added to a configuration of one embodiment. For example, a signal processing unit that has the configuration in Fig. 1 and the configuration in Fig. 2 in combination can be utilized. In addition, another configuration can be added to, deleted from and/or replaced with a part of a configuration of each embodiment.

Further, although the above-mentioned respective configurations, functions, processing units and so forth have been described with a focus on the example that the program for implementing some or all parts thereof is created, it goes without saying that, for example, the scan converter and so forth may be implemented by hardware by designing some or all parts thereof by, for example, an integrated circuit.

### Reference Signs List

- 11: probe
- 12: transmission/reception change-over SW
- 13: transmission BF
- 14: reception BF
- 15: control unit
- 16: Doppler image generation unit
- 17: B-image generation unit
- 18: cine memory
- 19: scan converter
- 20: display unit
- 21: structure detection unit
- 22: measurement region adjustment unit
- 23: elasticity evaluation unit
- 24: signal processing unit
- 51, 72, 82, 92, 132, 161: inspection object
- 52, 73, 83, 93, 162, 193: structural object
- 53: wave front of shear wave before passing
- 54: wave front of shear wave after passing
- 71, 81, 91, 111, 131, 151, 172: B-image
- 74, 114: reverse image
- 75: inspection object of reverse image
- 76: structural object of reverse image
- 84: color image
- 85: inspection object of color image
- 86: structural object of color image
- 94: Doppler image
- 95: inspection object of Doppler image
- 96: structural object of Doppler image
- 112: ROI
- 113: extracted image
- 115: first mask
- 116: second mask
- 133: detected structural object
- 134: non-detected object
- 135, 152: structure map
- 171: display screen
- 173: structure map
- 174: region setting button
- 175: measurement region
- 181: measurement region not including structural object
- 182: measurement region including structural object
- 191: measurement region before adjustment
- 192: measurement region after adjustment
- 194: focal region before adjustment
- 195: focal region after adjustment
- 196: measurement region after adjustment
- 201: blood vessel image
- 202: blood vessel
- 203: distorted image
- 204: strain homogeneous region
- 205: structure map including complex information of blood vessel and strain
- 206: optimum measurement region
- 207: moderately appropriate measurement region
- 208: inappropriate measurement region

## Claims

1. An ultrasonic diagnostic apparatus, comprising:
a probe that transmits and receives an ultrasonic wave; and
a processing unit that transmits and receives the ultrasonic wave from the probe to an inspection object and processes reception data obtained from the inspection object,
wherein the processing unit
detects a tissue structure of the inspection object from the reception data obtained by transmitting and receiving the ultrasonic wave from the probe to the inspection object,
determines a measurement region on the basis of the detected the tissue structure, and
evaluates elasticity of the measurement region on the basis of reception data obtained by transmission and reception of an ultrasonic wave to the measurement region.

2. An ultrasonic diagnostic apparatus, comprising:
a probe that transmits and receives an ultrasonic wave; and
a processing unit that transmits and receives the ultrasonic wave from the probe to an inspection object and processes reception data obtained from the inspection object,
wherein the processing unit
detects a tissue structure of the inspection object from reception data obtained by transmitting and receiving a first ultrasonic wave from the probe to the inspection object,
determines a measurement region for detecting a shear wave velocity on the basis of the tissue structure detected,
transmits a second ultrasonic wave to the measurement region to make it generate a shear wave, and
calculates the shear wave velocity from reception data obtained by transmitting and receiving a third ultrasonic wave to the measurement region.

3. The ultrasonic diagnostic apparatus according to claim 2,
wherein the processing unit is provided with
a structure detection unit that detects the tissue structure,
a measurement region adjustment unit that adjusts the measurement region and
an elasticity evaluation unit that evaluates elasticity of the measurement region using the shear wave velocity measured in the measurement region.

4. The ultrasonic diagnostic apparatus according to claim 2, further comprising:
a display unit,
wherein the processing unit
creates a structure map showing a position of the tissue structure and displays the structure map on the display unit.

5. The ultrasonic diagnostic apparatus according to claim 3, further comprising:
a display unit,
wherein the processing unit
creates a structure map for determining a position and a size of the tissue structure and displays the structure map on the display unit.

6. The ultrasonic diagnostic apparatus according to claim 4,
wherein the processing unit
utilizes at least a reverse image that a brightness of a brightness mode (B) image generated on the basis of the reception data obtained by transmitting and receiving the first ultrasonic wave to the inspection object has been reversed, a color image or a Doppler image that the tissue structure has been colored and an image indicative of tissue elasticity/strain as the structure map.

7. The ultrasonic diagnostic apparatus according to claim 4,
wherein the processing unit
creates the structure map by filtering having a mask modeling on the shape of the tissue structure.

8. The ultrasonic diagnostic apparatus according to claim 4,
wherein the processing unit
calculates the structure map by correlation arithmetic operation by utilizing a plurality of pieces of the reception data to be obtained at spatially different positions or a plurality of pieces of the reception data to be obtained at the spatially same position and at different times.

9. The ultrasonic diagnostic apparatus according to claim 4,
wherein the processing unit
visually displays correctness of the measurement region by a difference in color, shades of color, transparency of color.

10. The ultrasonic diagnostic apparatus according to claim 4,
wherein the processing unit
limits the measurement region or shifts the measurement region to a position avoiding the tissue structure on the basis of the structure map in adjustment of the measurement region.

11. The ultrasonic diagnostic apparatus according to claim 3, further comprising:
a display unit,
wherein a region setting button for selecting to conduct setting of the measurement region automatically or manually can be displayed on the display unit.

12. The ultrasonic diagnostic apparatus according to claim 2,
wherein the measurement region adjustment unit
outputs alert information as an image or a sound in a case where the measurement region is inappropriate for elasticity evaluation.

13. An elasticity evaluation method of
detecting a tissue structure of the inspection object from reception data obtained by transmitting and receiving a first ultrasonic wave from a probe that transmits and receives an ultrasonic wave to an inspection object;
determining a measurement region for detecting a shear wave velocity on the basis of the tissue structure detected;
transmitting a second ultrasonic wave to the measurement region to make it generate a shear wave;
calculating the shear wave velocity from reception data obtained by transmitting and receiving a third ultrasonic wave to the measurement region; and
conducting an elasticity evaluation of the inspection object on the basis of the shear wave velocity.

14. The elasticity evaluation method according to claim 13,
wherein a structure map for determining a position and a size of the tissue structure is created and the structure map is displayed.

15. The elasticity evaluation method according to claim 14, wherein
a reverse image that brightness of a B-image created on the basis of the reception data obtained by transmitting and receiving the first ultrasonic wave to the inspection object has been reversed, a color image that the tissue structure has been colored or a Doppler image is utilized as the structure map.

16. The elasticity evaluation method according to claim 14, wherein the structure map is created by filtering having a mask modeling on the shape of the tissue structure.

17. The elasticity evaluation method according to claim 14, wherein the structure map is calculated by correlation arithmetic operation by utilizing a plurality of pieces of the reception data to be acquired at spatially different positions or a plurality of pieces of the reception data to be acquired at the spatially same position and at different times.
